(19) Europäisches Patentamt
European Patent Office
Office européen des brevets

(11) Veröffentlichungsnummer: **0 166 186**
**A2**

(12) # EUROPÄISCHE PATENTANMELDUNG

(21) Anmeldenummer: **85106146.5**

(22) Anmeldetag: **20.05.85**

(51) Int. Cl.⁴: **C 07 C 69/736,** C 07 C 67/347, C 07 C 59/72, C 07 C 161/03, C 07 C 121/75, C 07 C 103/26, C 07 C 103/76, C 07 C 147/14, A 01 N 37/10, A 01 N 37/18, C 07 C 67/303 // C07C87/50

(30) Priorität: **28.05.84 DE 3419952**

(43) Veröffentlichungstag der Anmeldung: **02.01.86 Patentblatt 86/1**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Kirsten, Rolf, Dr., Carl-Langhans-Strasse 27, D-4019 Monheim (DE)**
Erfinder: **Wegler, Richard, Prof. Dr., Auf dem Forst 2, D-5090 Leverkusen 1 (DE)**
Erfinder: **Eue, Ludwig, Dr., Paul-Klee-Strasse 36, D-5090 Leverkusen 1 (DE)**
Erfinder: **Santel, Hans-Joachim, Dr., Gerstenkamp 19, D-5000 Köln 80 (DE)**
Erfinder: **Schmidt, Rudolf R., Dr., Im Waldwinkel 110, D-5060 Bergisch-Gladbach 2 (DE)**

(54) **Substituierte Phenoxyphenylpropionsäure-Derivate.**

(57) Neue substituierte Phenoxyphenylpropionsäure-Derivate der Formel

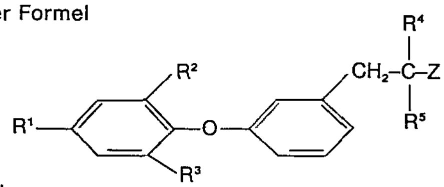

(I)

in welcher

$R^1$ für Halogen, Trihalogenmethylsulfonyl oder Trihalogenmethyl steht,

$R^2$ und $R^3$ unabhängig voneinander für Wasserstoff, Chlor oder Brom stehen,

$R^4$ und $R^5$ unabhängig voneinander für Wasserstoff, Cyano, Thiocyano, Chlor oder Brom stehen und

Z für Cyano oder für den Rest der Formel

CO–Y

steht, in welcher

Y für Hydroxy, Halogen, Alkoxy oder für den Rest der Formel

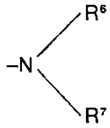

steht, in welcher

$R^6$ und 11 unabhängig voneinander für Wasserstoff, Alkyl mit 1 bis 4 Kohlenstoffatomen sowie für gegebenenfalls einfach bis dreifach substituiertes Phenyl stehen, mehrere Verfahren zur Herstellung der neuen Stoffe und deren Verwendung als Herbizide.

EP 0 166 186 A2

ACTORUM AG

BAYER AKTIENGESELLSCHAFT 5090 Leverkusen, Bayerwerk

Konzernverwaltung RP Dü/by-c
Patentabteilung Ib

## Substituierte Phenoxyphenylpropionsäure-Derivate

Die vorliegende Erfindung betrifft neue substituierte Phenoxyphenylpropionsäure-Derivate, mehrere Verfahren zu ihrer Herstellung und ihre Verwendung als Herbizide.

Es ist bereits bekannt, daß bestimmte Phenylpropionsäure-Derivate herbizide Eigenschaften besitzen (vgl. GB-PS 1 077 194). So kann z.B. 2-Chlor-3-(4-chlorphenyl)-propionsäuremethylester zur Unkrautbekämpfung eingesetzt werden. Die Wirkung dieser Verbindung ist gut, jedoch werden bei geringen Aufwandmengen einige Unkräuter nicht immer voll erfaßt.

Es wurden nun neue substituierte Phenoxyphenylpropionsäure-Derivate der Formel

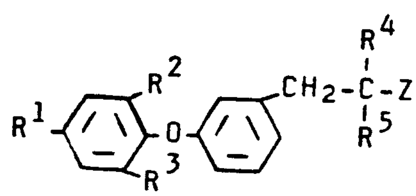

(I)

in welcher

$R^1$ für Halogen, Trihalogenmethylsulfonyl oder Trihalogenmethyl steht,

$R^2$ und $R^3$ unabhängig voneinander für Wasserstoff, Chlor oder Brom stehen,

$R^4$ und $R^5$ unabhängig voneinander für Wasserstoff, Cyano, Thiocyano, Chlor oder Brom stehen und

Z für Cyano oder für den Rest der Formel

CO-Y

steht, in welcher

Y für Hydroxy, Halogen, Alkoxy oder für den Rest der Formel

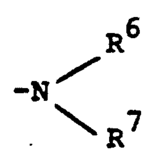

steht, in welcher

$R^6$ und $R^7$ unabhängig voneinander für Wasserstoff, Alkyl mit 1 bis 4 Kohlenstoffatomen sowie für gegebenenfalls einfach bis dreifach substituiertes Phenyl stehen,

gefunden.

Weiterhin wurde gefunden, daß man substituierte Phenoxyphenylpropionsäure-Derivate der Formel (I) erhält, wenn man

a) Phenoxyaniline der Formel

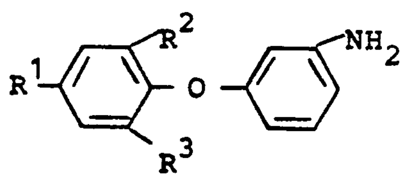

(II)

in welcher

$R^1$, $R^2$ und $R^3$ die oben angegebene Bedeutung haben,

oder Säureadditionssalze von Phenoxyanilinen der Formel (II) mit Diazotierungsmitteln in Gegenwart von Halogenwasserstoffsäuren der Formel

HX (III)

in welcher

X für Chlor oder Brom steht,

und in Gegenwart eines Verdünnungsmittels umsetzt und die dabei entstehenden Diazoniumsalze der Formel

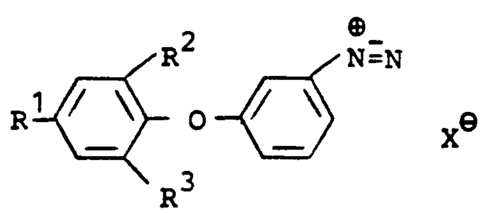

(IV)

in welcher

$R^1$, $R^2$, $R^3$ und X die oben angegebene Bedeutung haben,

mit Acrylsäure-Derivaten der Formel

$$CH_2=\underset{R^8}{\underset{|}{C}}-Z^1 \qquad (V)$$

in welcher

$R^8$ für Wasserstoff, Chlor oder Brom steht und

$Z^1$ für Cyano oder den Rest der Formel $-CO-Y^1$ steht,

in welcher

$Y^1$ für Hydroxy, Alkoxy oder für den Rest der Formel

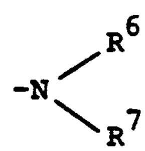

steht, worin

$R^6$ und $R^7$ die oben angegebene Bedeutung haben,

in Gegenwart von Halogenwasserstoffsäuren der Formel

$$HR^9 \qquad (VI)$$

in welcher

$R^9$ für Chlor oder Brom steht,

in Gegenwart eines Katalysators und gegebenenfalls in Gegenwart eines Verdünnungsmittels umsetzt und gegebenenfalls dabei entstehende Ester oder Nitrile in Gegenwart eines Verdünnungsmittels verseift und gegebenenfalls anschließend dabei entstehende substituierte Phenoxyphenylpropionsäure-Derivate der Formel

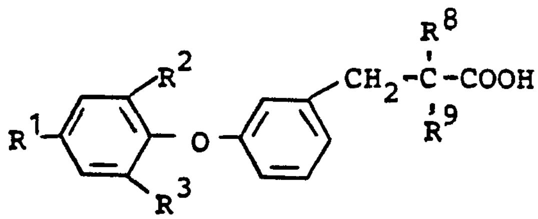

(Ia)

in welcher

$R^1$,$R^2$,$R^3$,$R^8$ und $R^9$ die oben angegebene Bedeutung haben,

gegebenenfalls in Gegenwart eines Verdünnungsmittels halogeniert,

oder

b) substituierte Phenoxyphenylpropionsäure-Derivate der Formel

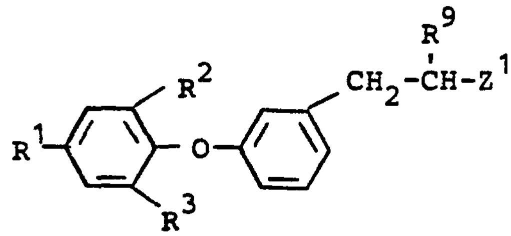

(Ib)

in welcher

$R^1$, $R^2$, $R^3$, $R^9$ und $Z^1$ die oben angegebene Bedeutung haben,

mit Alkalicyaniden oder Alkalirhodaniden gegebenenfalls in Gegenwart eines Verdünnungsmittels umsetzt,

oder

c) substituierte Phenoxyphenylpropionsäure-Derivate der Formel

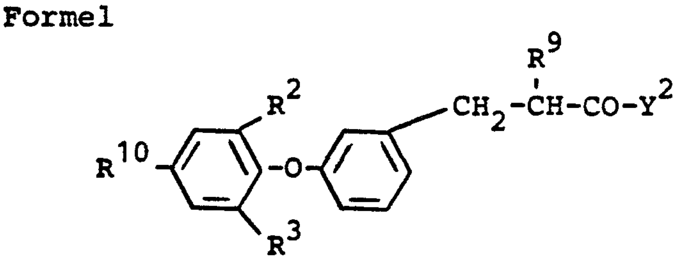

(Ic)

in welcher

$R^2$, $R^3$ und $R^9$ die oben angegebene Bedeutung haben,

$R^{10}$ für Halogen oder Trihalogenmethyl steht und

$Y^2$ für Hydroxy oder Alkoxy steht,

in Gegenwart eines Hydrierungskatalysators mit Wasserstoff gegebenenfalls in Gegenwart eines Verdünnungsmittels umsetzt,

oder

d) Phenoxyphenylpropensäure-Derivate der Formel

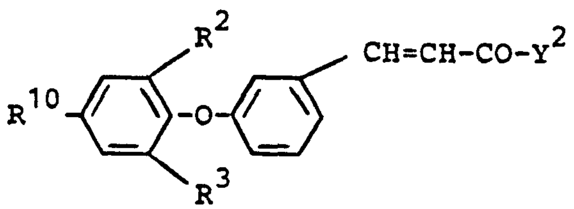

(VII)

in welcher

$R^2$,$R^3$,$R^{10}$ und $Y^2$ die oben angegebene Bedeutung haben,

mit Wasserstoff in Gegenwart eines Hydrierungskatalysators und gegebenenfalls in Gegenwart eines Verdünnungsmittels umsetzt.

Schließlich wurde gefunden, daß sich die neuen substituierten Phenoxyphenylpropionsäure-Derivate der Formel (I) durch hervorragende herbizide Wirksamkeit auszeichnen.

Überraschenderweise besitzen die erfindungsgemäßen substituierten Phenoxyphenylpropionsäure-Derivate der Formel (I) wesentlich bessere herbizide Eigenschaften als der aus dem Stand der Technik bekannte 2-Chlor-3-(4-chlorphenyl)-propionsäuremethylester, welcher ein hoch wirksamer Wirkstoff gleicher Wirkungsart ist. Vor allem lassen sich mit Hilfe der erfindungsgemäßen Wirkstoffe einige Ungräser, die von dem 2-Chlor-3-(4-chlorphenyl)-propionsäuremethylester nicht voll erfaßt werden, wirksam bekämpfen.

Die erfindungsgemäßen substituierten Phenoxyphenylpropionsäure-Derivate sind durch die Formel (I) eindeutig definiert. In dieser Formel steht $R^1$ vorzugsweise für Chlor, Trifluormethyl, Chlordifluormethyl oder Trifluormethylsulfonyl. $R^2$ und $R^3$ stehen unabhängig voneinander vorzugsweise für Wasserstoff, Chlor oder Brom. $R^4$ und $R^5$ stehen unabhängig voneinander vorzugsweise für Wasserstoff, Cyano, Thiocyano, Chlor oder Brom. Z steht vorzugsweise für Cyano oder den Rest der Formel -CO-Y, in welcher Y vorzugsweise für Hydroxy, Chlor, Brom, Alkoxy mit 1 bis 6 Kohlenstoffatomen oder für den Rest der Formel

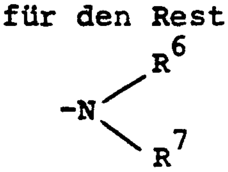

steht, in welcher $R^6$ und $R^7$ unabhängig voneinander vorzugsweise für Wasserstoff, Alkyl mit 1 bis 4 Kohlenstoffatomen oder für gegebenenfalls einfach

bis dreifach, gleichartig oder verschieden durch Methyl, Ethyl, Methoxy, Methylthio, Trifluormethyl, Trifluormethoxy, Trifluormethylthio, Fluor, Chlor und/oder Nitro substituiertes Phenyl stehen.

Besonders bevorzugt sind Verbindungen der Formel (I), in denen

$R^1$ für Chlor, Trifluormethyl oder Trifluormethylsulfonyl steht,

$R^2$ und $R^3$ unabhängig voneinander für Wasserstoff oder Chlor stehen,

$R^4$ und $R^5$ unabhängig voneinander für Wasserstoff, Cyano, Thiocyano oder Chlor stehen und

Z für Cyano oder den Rest der Formel -CO-Y steht, worin

Y für Hydroxy, Chlor, Brom, Methoxy, Ethoxy, n-Propoxy, iso-Propoxy, n-Butoxy, iso-Butoxy, sek.-Butoxy, tert.-Butoxy oder für den Rest der Formel

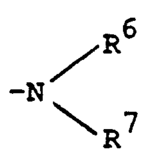

steht, worin

$R^6$ und $R^7$ unabhängig voneinander für Wasserstoff, Methyl, Ethyl, n-Propyl, iso-Propyl, n-Butyl, iso-Butyl oder für gegebenenfalls einfach bis dreifach durch Methyl, Methoxy, Trifluormethyl, Trifluormethoxy und/oder Chlor substituiertes Phenyl stehen.

Eine weitere Gruppe besonders bevorzugter erfindungsgemäßer Stoffe sind die substituierten Phenoxyphenylpropionsäure-Derivate der Formel (I), in denen

$R^1$ für Trifluormethylsulfonyl steht,

$R^2$ und $R^3$ unabhängig voneinander für Wasserstoff oder Chlor stehen,

$R^4$ und $R^5$ unabhängig voneinander für Wasserstoff oder Chlor stehen und

Z für Cyano oder den Rest der Formel -CO-Y steht, wobei

Y für Hydroxy, Methoxy, Ethoxy, n-Propoxy oder iso-Propoxy steht.

Eine weitere Gruppe von besonders bevorzugten erfindungsgemäßen Stoffen sind diejenigen Verbindungen der Formel (I), in denen

$R^1$ für Trifluormethyl steht,

$R^2$ und $R^3$ unabhängig voneinander für Wasserstoff oder Chlor stehen,

$R^4$ und $R^5$ unabhängig voneinander für Wasserstoff, Thiocyano oder Chlor stehen und

Z für Cyano oder den Rest der Formel -CO-Y steht, worin

Y für Hydroxy, Chlor, Brom, Methoxy, Ethoxy, n-Propoxy, iso-Propoxy, n-Butoxy oder iso-Butoxy steht.

Eine weitere Gruppe von besonders bevorzugten erfindungsgemäßen Verbindungen sind diejenigen Stoffe der Formel (I), in denen

$R^1$ für Trifluormethyl steht,

$R^2$ und $R^3$ unabhängig voneinander für Wasserstoff oder Chlor stehen,

$R^4$ und $R^5$ unabhängig voneinander für Wasserstoff oder Chlor stehen und

Z für den Rest der Formel $-CO-N\genfrac{}{}{0pt}{}{R^6}{R^7}$ steht, in welcher

$R^6$ und $R^7$ unabhängig voneinander für Wasserstoff, Methyl, Ethyl, n-Propyl, i-Propyl, n-Butyl sowie für gegebenenfalls einfach bis zweifach durch Methyl, Methoxy, Chlor und/oder Trifluormethyl substituiertes Phenyl stehen.

Verwendet man 3-(2,6-Dichlor-4-trifluormethyl-phenoxy)-anilin als Ausgangsstoff, Natriumnitrit und Salzsäure als Reaktionskomponenten und setzt das dabei entstehende Diazoniumsalz mit Acrylsäuremethylester und Salzsäure in Gegenwart von Kupfer-(I)-chlorid und Kupfer-(II)-chlorid um, so kann der Verlauf des erfindungsgemäßen Verfahrens (a) durch das folgende Formelschema wiedergegeben werden:

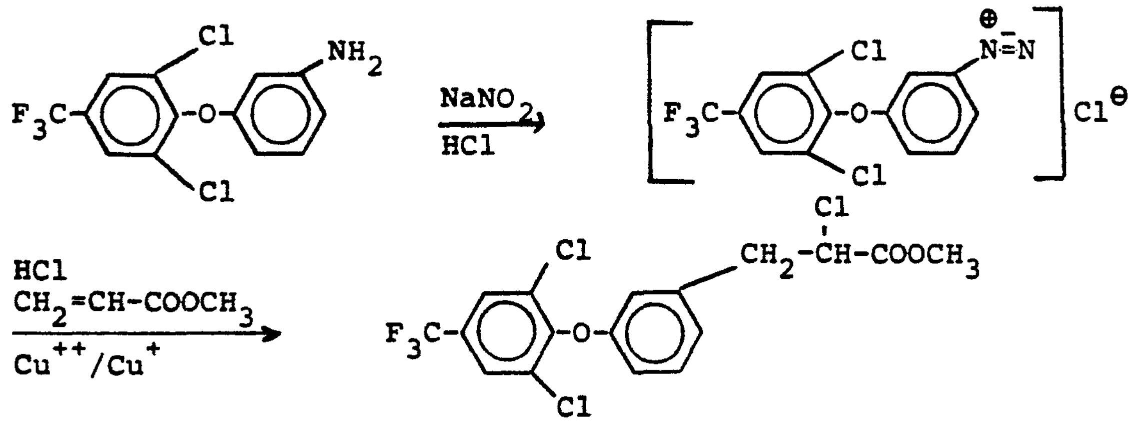


Verwendet man 2-Chlor-3-[3-(2,6-Dichlor-4-trifluormethyl-phenoxy)-phenyl]-propionsäuremethylester als Ausgangsstoff und Kaliumrhodanid als Reaktionskomponente, so kann der Verlauf des erfindungsgemäßen Verfahrens (b) durch das folgende Formelschema wiedergegeben werden:

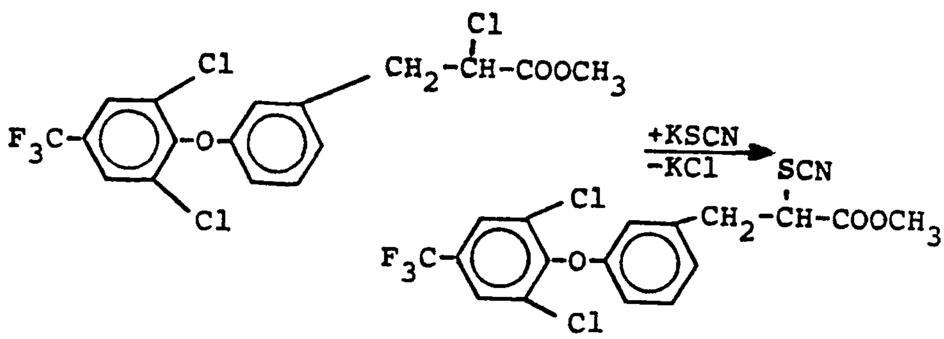


Verwendet man 2-Chlor-3-[3-(2-Chlor-4-trifluormethyl-phenoxy)-phenyl]-propionsäuremethylester als Ausgangsstoff und hydriert man mit Wasserstoff in Gegenwart von Raney-Nickel als Katalysator, so kann der Verlauf des erfindungsgemäßen Verfahrens (c) durch das folgende Formelschema wiedergegeben werden:

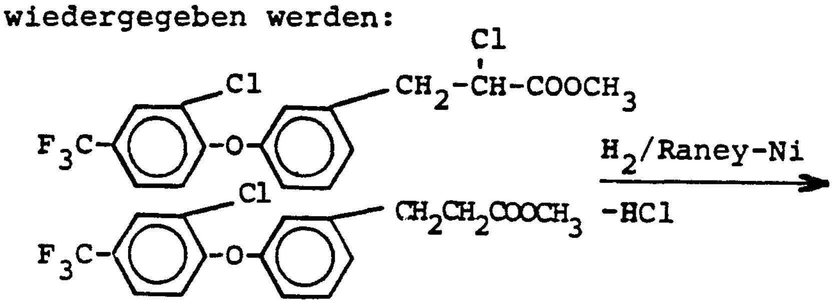


Verwendet man 3-[3-(2,6-Dichlor-4-trifluormethyl-phenoxy)-phenyl]-propensäuremethylester als Ausgangsstoff und hydriert man mit Wasserstoff in Gegenwart von Raney-Nickel als Katalysator, so kann der Verlauf des erfindungsgemäßen Verfahrens (d) durch das folgende Formelschema wiedergegeben werden:

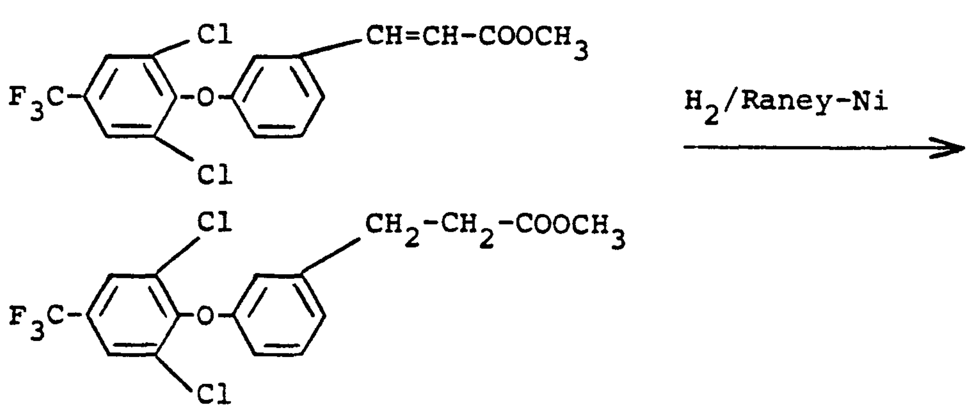

Die bei dem erfindungsgemäßen Verfahren (a) als Ausgangsstoffe benötigten Phenoxyaniline sind durch die Formel (II) definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden.

Als Säureadditionssalze der Phenoxyaniline der Formel (II) kommen vorzugsweise deren Hydrohalogenide, wie Hydrochloride oder Hydrobromide, in Frage.

Als Beispiele für die Verbindungen der Formel (II) seien genannt: 3-(4-Trifluormethylphenoxy)-, 3-(2-Chlor-4-trifluormethylphenoxy)-, 3-(2,6-Dichlor-4-trifluormethylphenoxy)-, 3-(2,4-Dichlor-phenoxy)-, 3-(2-Brom-4-trifluormethyl-phenoxy)-, 3-(4-Trifluormethylsulfonylphenoxy)-, 3-(2-Chlor-4-trifluormethylsulfonyl-phenoxy)-, 3-(2,6-Dichlor-4-trifluormethylsulfonyl-phenoxy)- und 3-(4-Chlordifluormethyl-phenoxy)-anilin und die entsprechenden Hydrochloride bzw. Hydrobromide.

Die Phenoxyaniline der Formel (II) und deren Säureadditionssalze sind bekannt oder lassen sich nach bekannten Methoden herstellen (vgl. DE-OS 26 52 810, EP-OS 0 029 128 und EP-OS 0 027 965 sowie Herstellungsbeispiele).

Die bei dem erfindungsgemäßen Verfahren (a) weiterhin als Ausgangsstoffe benötigten Acrylsäure-Derivate sind

durch die Formel (V) definiert. In dieser Formel steht $R^8$ für Wasserstoff, Chlor oder Brom. $Z^1$ steht vorzugsweise für Cyano oder den Rest der Formel $-CO-Y^1$, in welcher $Y^1$ vorzugsweise für Hydroxy, Alkoxy mit 1 bis 6 Kohlenstoffatomen oder für den Rest der Formel

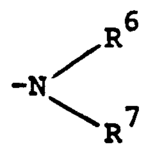

steht, in welcher $R^6$ und $R^7$ vorzugsweise für diejenigen Reste stehen, die bereits im Zusammenhang mit der Beschreibung der erfindungsgemäßen Stoffe der Formel (I) vorzugsweise für diese Reste genannt wurden.

Die Acrylsäure-Derivate der Formel (V) sind bekannte Verbindungen der organischen Chemie.

Als Katalysatoren können bei dem erfindungsgemäßen Verfahren (a) alle üblicherweise für derartige Umsetzungen mit Diazoniumsalzen verwendbaren Katalysatoren eingesetzt werden. Vorzugsweise in Frage kommen Kupfer-Pulver, Kupfer-I-chlorid und/oder Kupfer-II-chlorid.

Als Verdünnungsmittel kommen bei der Durchführung des erfindungsgemäßen Verfahrens (a) alle inerten, hydrophilen organischen Solventien und Wasser in Betracht. Vorzugsweise verwendbar sind Ether, wie Glykoldimethylether und Diglykoldimethylether, Tetrahydrofuran und

Dioxan, außerdem Ketone, wie Aceton, Methyl-ethyl-, Methyl-isopropyl- und Methyl-isobutyl-keton. Das erfindungsgemäße Verfahren (a) wird bevorzugt unter Verwendung von Gemischen aus organischen Solventien und Wasser durchgeführt.

Die Reaktionstemperaturen können bei der Umsetzung nach dem erfindungsgemäßen Verfahren (a) innerhalb eines größeren Bereichs variiert werden. Im allgemeinen arbeitet man bei Temperaturen zwischen -15°C und + 80°C, vorzugsweise zwischen 5°C und +60°C.

Das erfindungsgemäße Verfahren (a) wird im allgemeinen unter Normaldruck durchgeführt.

Zur Durchführung des erfindungsgemäßen Verfahrens (a), werden die Ausgangsstoffe der Formeln (II), (III), (V) und (VI) im allgemeinen in angenähert äquimolaren Mengen eingesetzt. Es ist jedoch auch möglich, eine der jeweils eingesetzten Komponenten in einem größeren Überschuß zu verwenden. Die Aufarbeitung erfolgt jeweils nach üblichen Verfahren.

Ist die Herstellung einer Verbindung der Formel (I) beabsichtigt, in der Z für COOH steht, so geht man im allgemeinen so vor, daß man einen entsprechenden Ester (Z = CO-Alkoxy) oder ein Nitril (Z = CN) verseift. Diese Verseifung erfolgt nach üblichen Methoden.

Als Verseifungsmittel kommen sowohl saure als auch basische Reagenzien in Betracht. Bei der basischen Verseifung sind wäßrige Alkalihydroxid-Laugen, wie z.B. Natronlauge oder Kalilauge, vorzugsweise verwendbar. Als saure Verseifungsreagenzien kommen vorzugsweise Mineralsäuren, wie z.B. Salzsäure, Bromwasserstoffsäure oder Schwefelsäure, in Form ihrer wäßrigen Lösungen oder gegebenenfalls auch als Gase in Frage.

Die Verseifung wird im allgemeinen in Gegenwart eines Verdünnungsmittels vorgenommen. Vorzugsweise in Betracht kommen aromatische Kohlenwasserstoffe, wie Toluol oder Xylol, ferner Alkohole, wie Methanol oder Ethanol, außerdem Ether, wie Dioxan und Tetrahydrofuran, und auch Gemische aus organischen Solventien und Wasser.

Bei der sauren Verseifung können als Verdünnungsmittel auch gegebenenfalls durch Halogen substituierte Carbonsäuren, wie z.B. Ameisensäure, Essigsäure, Chloressigsäure, Bromessigsäure, Propionsäure und Brompropionsäure eingesetzt werden.

Die Temperaturen können bei der Durchführung der Verseifung innerhalb eines größeren Bereiches variiert werden. Im allgemeinen arbeitet man bei Temperaturen zwischen 0°C und 160°C, vorzugsweise zwischen 20°C und 140°C.

Bei der Durchführung der Verseifung geht man im allgemeinen so vor, daß man den Ester oder das Nitril der Formel (I) in Gegenwart eines Verdünnungsmittels mit einer äquivalenten Menge oder einem Überschuß an Base oder Säure behandelt. Die Mineralsäure kann, z.B. bei Verwendung von Halogenwasserstoffsäuren, auch gasförmig durch die Lösung geleitet werden. Die Aufarbeitung erfolgt nach üblichen Methoden. Im allgemeinen geht man so vor, daß man das Reaktionsgemisch durch Abziehen des Lösungsmittels unter vermindertem Druck einengt, den verbleibenden Rückstand in Wasser aufnimmt, mit Mineralsäure, wie z.B. Salzsäure, ansäuert und die sich dabei abscheidende Säure der Formel (I) abtrennt.

Bei der Verseifung von Nitrilen (Z = CN) in Gegenwart von Alkoholen, wie z.B. Methanol oder Ethanol, entstehen die entsprechenden Ester der Formel (I).

Ist die Herstellung einer Verbindung der Formel (I) beabsichtigt, in der Z für -CO-Y steht, wobei Y für Halogen steht, so geht man zweckmäßigerweise so vor, daß man eine nach dem erfindungsgemäßen Verfahren (a) hergestellte Säure der Formel (Ia) mit einem Halogenierungsmittel, wie z.B. Thionylchlorid, Thionylbromid oder Phosphor-tribromid, gegebenenfalls in Gegenwart eines Katalysators, wie z.B. Dimethylformamid oder Triphenylphosphinoxid, in Gegenwart eines inerten Verdünnungsmittels, wie z.B. Methylenchlorid, Toluol oder 1,2-Dichlorethan, bei Temperaturen zwischen 10 und 140°C umsetzt. Die Aufarbeitung erfolgt nach üblichen Methoden.

Die beim erfindungsgemäßen Verfahren (b) als Ausgangsstoffe benötigten Verbindungen sind durch die Formel (Ib) definiert. In dieser Formel haben $R^1$, $R^2$ und $R^3$ vorzugsweise diejenigen Bedeutungen die bereits im Zusammenhang mit der Beschreibung der erfindungsgemäßen Stoffe der Formel (I) vorzugsweise für diese Reste genannt wurden. $R^9$ steht vorzugsweise für Chlor oder Brom. $Z^1$ steht vorzugsweise für Cyano oder für den Rest der Formel ($-CO-Y^1$, in welcher $Y^1$ vorzugsweise für Hydroxy, für Alkoxy mit 1 bis 6 Kohlenstoffatomen oder für den Rest der Formel

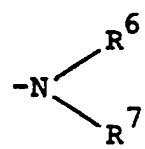

in welcher

$R^6$ und $R^7$ vorzugsweise diejenigen Bedeutungen haben, die bereits im Zusammenhang mit der Beschreibung der erfindungsgemäßen Stoffe der Formel (I) vorzugsweise für diese Reste genannt wurden.

Die Verbindungen der Formel (Ib) lassen sich nach dem erfindungsgemäßen Verfahren (a) herstellen.

Als Alkali-cyanide oder -rhodanide werden beim erfindungsgemäßen Verfahren (b) vorzugsweise Natrium- und Kaliumcyanid oder Natrium- und Kaliumrhodanid verwendet.

Als Verdünnungsmittel kommen bei der Durchführung des erfindungsgemäßen Verfahrens (b) alle inerten organischen Solventien in Betracht. Vorzugsweise verwendbar sind Nitrile, wie z.B. Acetonitril und Propionitril , Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Temperaturen können bei der Umsetzung nach dem erfindungsgemäßen Verfahren (b) innerhalb eines größeren Bereiches variiert werden. Im allgemeinen arbeitet man bei Temperaturen zwischen 0°C und 160°C, vorzugsweise zwischen 20°C und 140°C.

Das erfindungsgemäße Verfahren (b) wird im allgemeinen unter Normaldruck durchgeführt. Es ist jedoch auch möglich, unter erhöhtem oder vermindertem Druck zu arbeiten.

Bei der Durchführung des erfindungsgemäßen Verfahrens (b) geht man im allgemeinen so vor, daß man die Verbindungen der Formel (Ib) mit einer äquivalenten Menge oder mit einem Überschuß an Alkali-cyanid bzw. -rhodanid in Gegenwart eines Verdünnungsmittels bei der jeweils gewünschten Temperatur behandelt. Die Aufarbeitung geschieht nach üblichen Methoden.

Die beim erfindungsgemäßen Verfahren (c) als Ausgangsstoffe benötigten Verbindungen sind durch die Formel (Ic) definiert. In dieser Formel haben $R^2$ und $R^3$ vorzugsweise

diejenigen Bedeutungen, die bereits im Zusammenhang mit der Beschreibung der erfindungsgemäßen Stoffe der Formel (I) vorzugsweise für diese Reste genannt wurden. $R^9$ steht vorzugsweise für Chlor oder Brom. $R^{10}$ steht vorzugsweise für Chlor, Trifluormethyl oder Chlordifluormethyl.

$Y^2$ steht vorzugsweise für Hydroxy oder für Alkoxy mit 1 bis 6 Kohlenstoffatomen.

Die Verbindungen der Formel (Ic) lassen sich nach dem erfindungsgemäßen Verfahren (a) herstellen (vgl. oben).

Als Hydrierungskatalysatoren können bei dem erfindungsgemäßen Verfahren (c) alle üblicherweise für derartige Reaktionen verwendbaren Katalysatoren eingesetzt werden. Vorzugsweise verwendet man Raney-Nickel oder Edelmetallkatalysatoren wie Palladium, Palladiumoxid, Platin oder Platinoxid, gegebenenfalls auf einem geeigneten Trägerstoff, wie z.B. Kohle.

Als Verdünnungsmittel kommen bei der Durchführung des erfindungsgemäßen Verfahrens (c) alle unter Hydrierungsbedingungen inerten Solventien in Betracht. Vorzugsweise verwendbar sind Kohlenwasserstoffe wie Pentan, Hexan, Heptan, Cyclohexan und Alkohole wie Methanol, Ethanol, n-Propanol und i-Propanol.

Die Temperaturen können bei der Umsetzung nach dem erfindungsgemäßen Verfahren (c) innerhalb eines größeren

Bereichs variiert werden. Im allgemeinen arbeitet man bei Temperaturen zwischen 0°C und 100°C, vorzugsweise zwischen 20°C und 80°C.

Das erfindungsgemäße Verfahren (c) wird unter erhöhtem Druck in Gegenwart von Wasserstoff durchgeführt. Im allgemeinen arbeitet man bei Druckverhältnissen zwischen 40 atm und 140 atm, vorzugsweise zwischen 60 und 120 atm.

Bei der Durchführung des erfindungsgemäßen Verfahrens (c) geht man im allgemeinen so vor, daß man die Verbindungen der Formel (Ic) in Gegenwart eines geeigneten Katalysators und in Gegenwart eines Verdünnungsmittels, bei der jeweils gewünschten Temperatur mit Wasserstoff im Autoklaven umsetzt. Die Aufarbeitung geschieht nach üblichen Methoden.

Die beim erfindungsgemäßen Verfahren (d) als Ausgangsstoffe benötigten Phenoxyphenylpropensäure-Derivate sind durch die Formel (VII) definiert. In dieser Formel haben $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden. $R^{10}$ steht vorzugsweise für Chlor, Trifluormethyl oder Chlordifluormethyl. $Y^2$ steht vorzugsweise für Hydroxy oder für Alkoxy mit 1 bis 6 Kohlenstoffatomen.

Die Verbindungen der Formel (VII) sind bekannt und lassen sich auf einfache Weise nach bekannten Methoden herstellen (vgl. z.B. DE-OS 3 044 810 und die Herstellungsbeispiele).

Bei dem erfindungsgemäßen Verfahren (d) entsprechen die Umsetzungsbedingungen denen des erfindungsgemäßen Verfahrens (c) (vgl. oben).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können insbesondere zur selektiven Bekämpfung von unerwünschtem Pflanzenwuchs in monokotylen Kulturen, wie z.B. Getreide, Reis, Mais und Zuckerrohr, sowie in dikotylen Kulturen, wie z.B. Zuckerrüben, eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und

Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch insbesondere nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

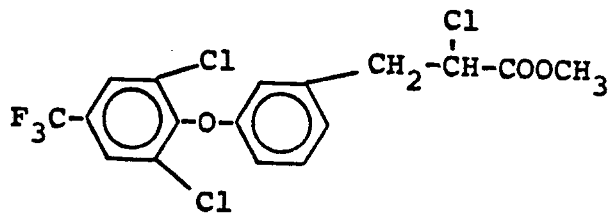

(I-1)

(Verfahren a))

200 g (0,468 Mol) 3-(2,6-Dichlor-4-trifluormethylphenoxy)-anilin wurden in einem Gemisch aus 600 ml Aceton und 300 ml konzentrierter wäßriger Salzsäure bei 0°C bis 10°C durch Zutropfen von 150 ml 30 %iger wäßriger Natriumnitritlösung diazotiert. Dann wurde das Reaktionsgemisch mit 80 ml (0,887 Mol) Acrylsäuremethylester versetzt. Unter Rühren wurde eine Lösung von 9 g Kupfer(I)chlorid und 3 g Kupfer(II)chlorid in 145 ml halbkonzentrierter wäßriger Salzsäure zugetropft. Das Reaktionsgemisch wurde noch 45 Minuten bei 20°C gerührt, anschließend mit Wasser verdünnt und mit Diethylether extrahiert.

Die Etherphase wurde mit 5 %iger wäßriger Natriumhydrogencarbonatlösung gewaschen, über Natriumsulfat getrocknet und eingeengt. Man erhielt nach chromatographischer Reinigung mit Methylenchlorid als Laufmittel über eine Kieselgelsäule 211 g (76 % der Theorie) an 2-Chlor-3-[3-(2,6-dichlor-4-trifluormethyl-phenoxy)-phenyl]-propionsäuremethylester als gelbes Öl.

$^1H$-NMR ($CDCl_3$): $\delta$ = 3,15 (d,d, 1H, $-CH_2-$), 3,35 (d,d, 1H, $-CH_2-$), 4,44 (quasi-t, 1H, -CHCl) ppm.

Beispiel 2

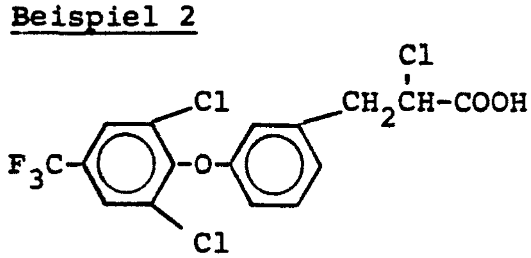

(I-2)

(Verfahren a))

30 g (0,07 Mol) 2-Chlor-3-/2-(2,6-dichlor-4-trifluormethyl-phenoxy)-phenyl/-propionsäuremethylester wurden in 200 ml Ameisensäure bei ständigem Durchleiten von Chlorwasserstoffgas 15 Stunden unter Rückfluß erhitzt. Nach dem Abdestillieren des Lösungsmittels im Vakuum wurde der Rückstand in 300 ml Wasser aufgenommen und mit 500 ml Methylenchlorid extrahiert. Die organische Phase wurde über Natriumsulfat getrocknet, filtriert und eingeengt. Der Rückstand wurde anschließend aus n-Hexan umkristallisiert. Man erhielt 19,4 g (66,8 % der Theorie) an 2-Chlor-3-/3-(2,6-dichlor-4-trifluormethyl-phenoxy)-phenyl/-propionsäure in Form farbloser Kristalle vom Schmelzpunkt 152 bis 154°C.

Beispiel 3

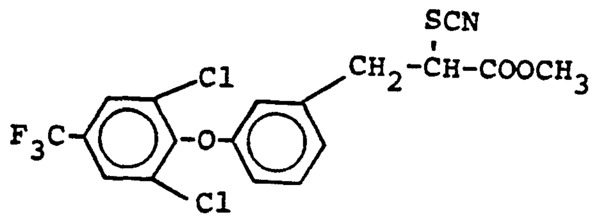

(I-3)

(Verfahren b))

Ein Gemisch aus 8,6 g (0,02 Mol) 2-Chlor-3-/3-(2,6-dichlor-4-trifluormethyl-phenoxy)-phenyl7-propionsäuremethylester, 80 ml Dimethylsulfoxid und 3 g Kaliumrhodanid wurde 6 Stunden bei 100°C gerührt. Das Reaktionsgemisch wurde anschließend mit 500 ml Wasser verrührt und mit 400 ml Diethylether extrahiert. Die organische Phase wurde über Natriumsulfat getrocknet, filtriert und eingeengt. Der Rückstand wurde im Vakuum mit einer Kugelrohrdestillationsapparatur destilliert. Man erhielt 1,9 g (21 % der Theorie) an 3-/3-(2,6-Dichlor-4-trifluormethyl-phenoxy)-phenyl7-2-thiocyano-propionsäuremethylester als nahezu farbloses Öl.

$^1$H-NMR ($CDCl_3$): $\delta$ = 3,22 (d,d, 1H, $-CH_2$), 3,40 (d,d, 1H, $-CH_2$), 3,95 (m, 1H, -CH-) ppm.

Beispiel 4

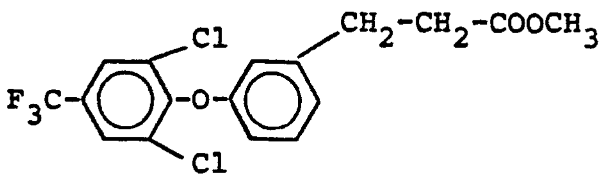

(I-4)

(Verfahren c)

6,4 g (0,015 Mol) 2-Chlor-3-/3-(2,6-dichlor-4-trifluormethyl-phenoxy)-phenyl/-propionsäuremethylester wurden in 100 ml Methanol bei 40°C und 100 atm Wasserstoffdruck in Gegenwart von 1,5 g Raney-Nickel hydriert. Nach dem Abfiltrieren des Katalysators wurde das Filtrat eingeengt. Man erhielt 4,7 g (80 % der Theorie) 3-/3-(2,6-Dichlor-4-trifluormethyl-phenoxy)-phenyl/-propionsäuremethylester vom Schmelzpunkt 58 bis 60°C.

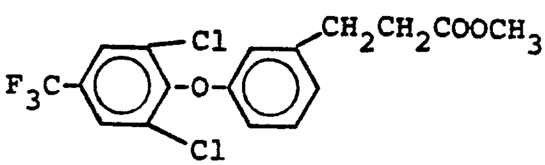

(I-4)

(Verfahren d)

8,5 g (0,0217 Mol) 3-/3-(2,6-Dichlor-4-trifluormethyl-phenoxy)-phenyl/-propensäuremethylester wurden in 150 ml Methanol bei 50 bis 60°C unter 85 atm Wasserstoffdruck in Gegenwart von 1 g Raney-Nickel hydriert. Nach dem Abfiltrieren des Katalysators wurde das Filtrat eingeengt. Man erhielt 8,3 g (97,3 % der Theorie) an 3-/3-(2,6-Dichlor-4-trifluormethyl-phenoxy)-phenyl7-propionsäuremethylester.
Schmelzpunkt: 58-60°C.

Beispiel 5

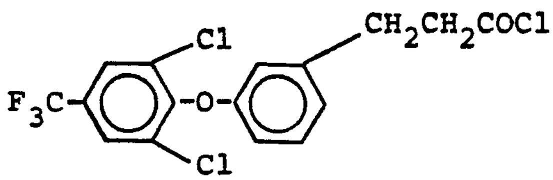

(I-5)

(Verfahren a)

Ein Gemisch aus 15 g (0,0396 Mol) 3-/3-(2,6-Dichlor-4-trifluormethyl-phenoxy)-phenyl7-propionsäure und 150 ml Thionylchlorid wurde 5 1/2 Stunden unter Rückfluß erhitzt. Danach wurde der Überschuß an Thionylchlorid unter vermindertem Druck entfernt. Man erhielt 14,8 g (94 % der Theorie) an 3-/3-(2,6-Dichlor-4-trifluormethyl-phenoxy)-phenyl7-propionsäurechlorid.

$^1H$-NMR ($CDCl_3$): $\delta$ = 2,98 (t, 2H, $-CH_2$), 3,18 (t, 2H, $-CH_2-CO-$) ppm.

Beispiel 6

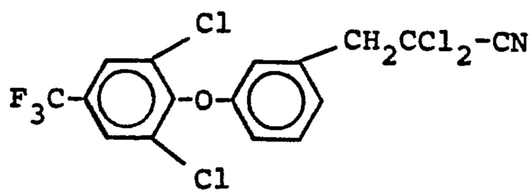

(I-6)

(Verfahren a)

17,9 g (0,05 Mol) 3-(2,6-Dichlor-4-trifluormethyl-phenoxy)-anilin-hydrochlorid wurden in einem Gemisch aus 100 ml Aceton und 50 ml konzentrierter Salzsäure bei 0 bis 10°C mit 25 ml 30 %iger wäßriger Natriumnitritlösung diazotiert. Dann fügte man 6 ml (0,075 Mol) 2-Chloracrylnitril zu. Unter Rühren wurde eine Lösung von 0,9 g Kupfer(I)chlorid und 0,3 g Kupfer(II)chlorid in 15 ml halbkonzentrierter wäßriger Salzsäure zugetropft. Nachdem die Stickstoffentwicklung eingesetzt hatte, wurde das Reaktionsgemisch noch 45 Minuten bei 20°C und anschließend 30 Minuten bei 40°C gerührt. Dann wurde das Reaktionsgemisch mit Wasser verdünnt und ausgeethert. Die Etherphase wurde abgetrennt, mit 5 %iger wäßriger Natriumhydrogencarbonatlösung gewaschen und über Natriumsulfat getrocknet. Die etherische Lösung wurde filtriert und eingedampft, und der verbleibende Rückstand wurde mit Methylenchlorid als Laufmittel über eine Kieselgelsäule chromatographiert. Man erhielt so 13,2 g rohes 2,2-Dichlor-3-/3-(2,6-dichlor-4-trifluormethyl-phenoxy)-phenyl/-propionitril,

das nach dem Umkristallisieren aus n-Hexan 7,7 g (35,9 % der Theorie) farblose Kristalle vom Schmelzpunkt 99 bis 100°C ergab.

Beispiel 7

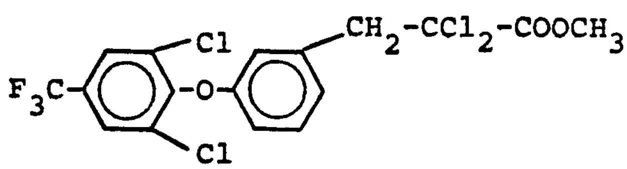

(I-7)

(Verfahren a)

4 g (0,0093 Mol) 2,2-Dichlor-3-[3-(2,6-dichlor-4-trifluormethyl-phenoxy)-phenyl]-propionitril wurden in einem Gemisch aus 75 ml Methanol und 75 ml Diethylether gelöst, und die Lösung wurde bei 0 bis 5°C mit Chlorwasserstoffgas gesättigt. Anschließend wurde das Reaktionsgemisch 14 Stunden bei 20°C gerührt. Danach fügte man 200 ml Wasser hinzu und leitete zur Vertreibung der Salzsäure Luft durch die Lösung. Das Reaktionsgemisch wurde dann mit Ether extrahiert. Die Etherphase wurde abgetrennt und mit 5 %iger wäßriger Natriumhydrogencarbonatlösung gewaschen, über Natriumsulfat getrocknet, filtriert und eingeengt. Der Rückstand wurde mit n-Heptan zur Kristallisation gebracht. Man erhielt 3,4 g (79,1 % der Theorie) an 2,2-Dichlor-3-[3-(2,6-dichlor-4-trifluormethyl-phenoxy)-phenyl]-propionsäuremethylester vom Schmelzpunkt 123 bis 130°C.

Analog den Beispielen 1 bis 7 bzw. den Verfahrensvarianten a) bis d) wurden die in den folgenden Beispielen aufgeführten Verbindungen der Formel (I) hergestellt.

Beispiel 8

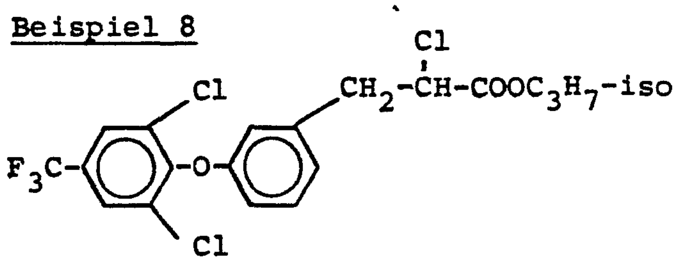

(I-8)

$^1$H-NMR ($CDCl_3$): $\delta$ = 3,34 (d,d, 1H, $-CH_2-$), 3,32 (d,d, 1H, $-CH_2-$), 4,36 (quasi-t, 1H, -CHCl-) ppm.

Beispiel 9

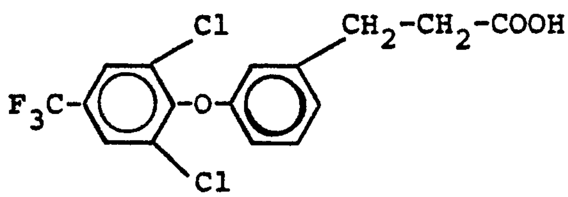

(I-9)

$^1$H-NMR ($CDCl_3$): $\delta$ = 2,65 (t, 2H, $-CH_2-$), 2,92 (t, 2H, $-CH_2-CO-$) ppm.

Beispiel 10

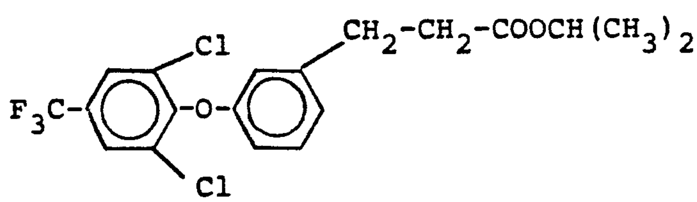

(I-10)

$^{1}$H-NMR ($CDCl_3$): $\delta$ = 2,55 (t, 2H, $-CH_2-$), 2,90 (t, 2H, $-CH_2-CO-$) ppm.

Beispiel 11

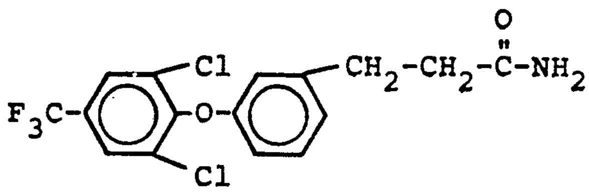

(I-11)

$^{1}$H-NMR ($CDCl_3$): $\delta$ = 2,51 (t, 2H, $-CH_2-$), 2,94 (t, 2H, $-CH_2-CO-$) ppm.

Beispiel 12

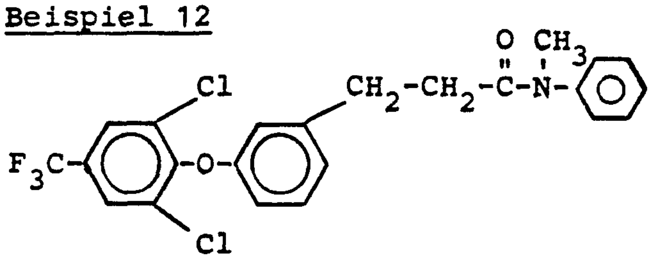

(I-12)

$^{1}$H-NMR ($CDCl_3$): $\delta$ = 2,35 (t, 2H, $-CH_2-$), 2,87 (t, 2H, $-CH_2-CO-$) ppm.

Beispiel 13

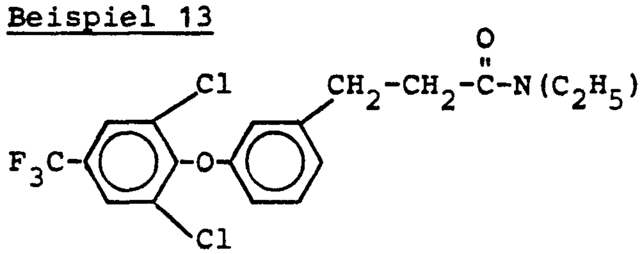

(I-13)

$^{1}$H-NMR ($CDCl_3$): $\delta$ = 2,57 (t, 2H, $-CH_2-$), 2,96 (t, 2H, $-CH_2-CO-$) ppm.

Beispiel 14

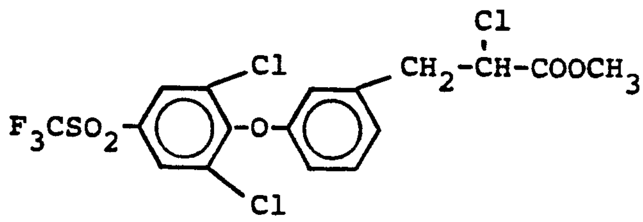

(I-14)

$^1$H-NMR ($CDCl_3$): $\delta$ = 3,16 (d,d, 1H, $-CH_2-$), 3,36 (d,d, 1H, $-CH_2-$), 4,42 (quasi-t, 1H, -CHCl-) ppm.

Ausgangsprodukte der Formel (II)

Beispiel 15

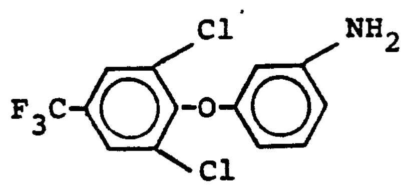

(II-1)

65 g (0,6 Mol) 3-Aminophenol wurden mit 28 g (0,7 Mol) Natriumhydroxid in 500 ml Dimethylsulfoxid 30 Minuten bei 120°C gerührt. Anschließend wurden 150 g 3,4,5-Trichlor-1-trifluormethyl-benzol zugetropft und es wurde 5 Stunden bei 120°C gerührt. Nach dem Abkühlen auf 25°C wurde das Reaktionsgemisch in 1 l Wasser eingerührt und mit Methylenchlorid extrahiert.

Die organische Phase wurde getrocknet, filtriert und eingeengt. Man erhielt 200 g rohes 3-(2,6-Dichlor-4-trifluormethyl-phenoxy)-anilin. Zur Reinigung wurde

das Produkt in 100 ml Methanol eingerührt. Es wurden 400 ml konzentrierte wäßrige Salzsäure und 200 ml Wasser zugegeben, danach wurde abgesaugt und bei 30°C im Vakuum getrocknet. Auf diese Weise wurden 192 g des Hydrochlorids von 3-(2,6-Dichlor-4-trifluormethyl-phenoxy)-anilin vom Schmelzpunkt 200 bis 204°C erhalten.

Ausgangsprodukte der Formel (VII)

Beispiel 16

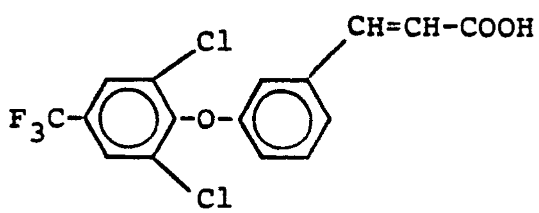

(VII-1)

83,8 g (0,23 Mol) 3-(2,6-Dichlor-4-trifluormethyl-phenoxy)-benzaldehyd wurden mit 32,5 g (0,312 Mol) Malonsäure und 500 g Natriumacetat in 350 ml Eisessig 6 1/2 Stunden unter Rückfluß erhitzt. Dann ließ die Kohlendioxid-Entwicklung nach. Es wurden noch 16,3 g (0,156 Mol) Malonsäure und 25 g Natriumacetat hinzugegeben und das Reaktionsgemisch wurde weitere 8 Stunden bei 125°C gerührt bis die Kohlendioxid-Entwicklung beendet war. Das Reaktionsgemisch wurde anschließend bei 90°C in 1 l Wasser eingegossen und 10 Minuten nachgerührt. Das Reaktionsprodukt wurde abgesaugt, mit 500 ml Wasser nachgewaschen und bei 80°C getrocknet. Das Reaktionsprodukt wurde in 300 ml

n-Hexan unter Rückfluß erhitzt, abgesaugt, mit 200 ml n-Hexan nachgewaschen und bei 80°C getrocknet.

Man erhielt 73,7 g (78 % der Theorie) an 3-[3-(2,6-Dichlor-4-trifluormethyl-phenoxy)-phenyl]-propensäure vom Schmelzpunkt 180 bis 181°C.

Verwendungsbeispiel

In dem nachfolgenden Verwendungsbeispiel wurde die folgende Verbindung als Vergleichssubstanz eingesetzt:

(A) = 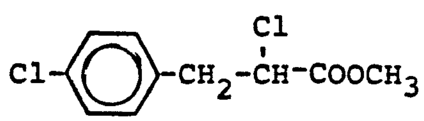

2-Chlor-3-(4-chlorphenyl)-propionsäuremethylester

(bekannt aus GB-PS 1 077 194).

Beispiel A

Post-emergence-Test/Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Wirkung

Wirkstoffe, Wirkstoffkonzentrationen und Versuchsergebnisse gehen aus der folgenden Tabelle hervor.

**Tabelle A**

Post-emergence Test / Gewächshaus

| Wirkstoff | Wirkstoff-aufwand kg/ha | Zucker-rüben | Weizen | Galinsoga | Sinapis | Portulak | Solanum | Amaranthus |
|---|---|---|---|---|---|---|---|---|
| 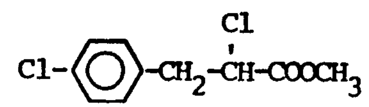 | 0,5 | 0 | 0 | 0 | 0 | 0 | 0 | 20 |
| (bekannt) (A) 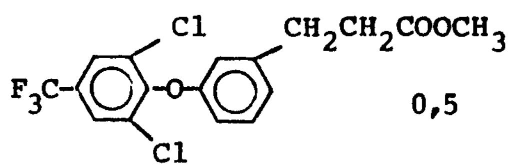 | 0,5 | 0 | 0 | 100 | 100 | 100 | 100 | 100 |
| (I-4) 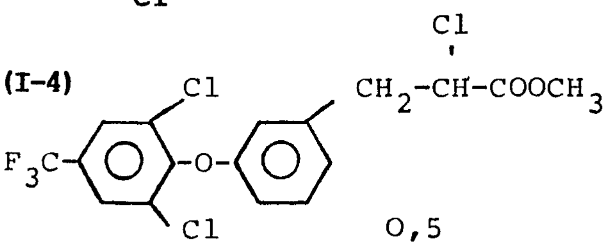 (I - 1) | 0,5 | 0 | 0 | 40 | 90 | 100 | 100 | 100 |

Tabelle A ( Fortsetzung) Post-emergence Test / Gewächshaus

| Wirkstoff | Wirkstoff-aufwand kg/ha | Zucker-rüben | Weizen | Galin-soga | Sinapis | Portu-lak | Solanum | Ama-ranthus |
|---|---|---|---|---|---|---|---|---|
| 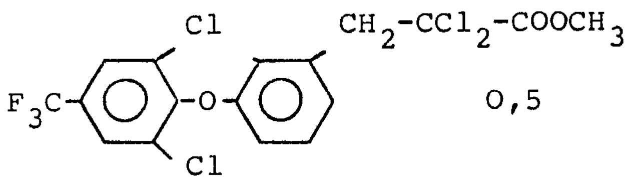 (I-7) | 0,5 | 20 | 0 | 70 | 95 | 80 | 100 | 100 |
| 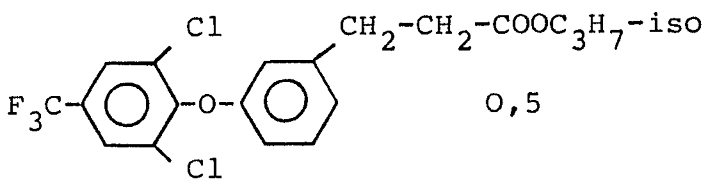 (I-1o) | 0,5 | 45 | 10 | 100 | 100 | 50 | 95 | 100 |

Beispiel B

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
++ starkes Austrocknen der Blätter, starker Blattfall
+++ sehr starkes Austrocknen der Blätter, sehr starker Blattfall

Wirkstoffe, Wirkstoffkonzentration und Versuchsergebnisse gehen aus der folgenden Tabelle hervor.

**Tabelle B**

**Entlaubung und Austrocknung der Blätter bei Baumwolle**

| Wirkstoff | Wirkstoffkonzentration in % | Wirkung |
|---|---|---|
| (Kontrolle) | - | = 0 |
| (I - 1) | 0,05 | +++ |

Patentansprüche

1. Substituierte Phenoxyphenylpropionsäure-Derivate der Formel

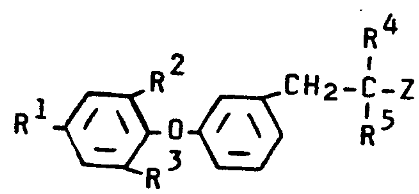

(I)

in welcher

$R^1$ für Halogen, Trihalogenmethylsulfonyl oder Trihalogenmethyl steht,

$R^2$ und $R^3$ unabhängig voneinander für Wasserstoff, Chlor oder Brom stehen,

$R^4$ und $R^5$ unabhängig voneinander für Wasserstoff, Cyano, Thiocyano, Chlor oder Brom stehen und

Z für Cyano oder für den Rest der Formel

CO-Y

steht, in welcher

Y für Hydroxy, Halogen, Alkoxy oder für den Rest der Formel

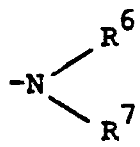

steht, in welcher

$R^6$ und $R^7$ unabhängig voneinander für Wasserstoff, Alkyl mit 1 bis 4 Kohlenstoffatomen sowie für gegebenenfalls einfach bis dreifach substituiertes Phenyl stehen.

2. Substituierte Phenoxyphenylpropionsäure-Derivate der Formel (I), in denen

$R^1$ für Chlor, Trifluormethyl, Chlordifluormethyl oder Trifluormethylsulfonyl steht,

$R^2$ und $R^3$ unabhängig voneinander für Wasserstoff, Chlor oder Brom stehen,

$R^4$ und $R^5$ unabhängig voneinander für Wasserstoff, Cyano, Thiocyano, Chlor oder Brom stehen und

Z für Cyano oder den Rest der Formel -CO-Y steht,

in welcher

Y für Hydroxy, Chlor, Brom, Alkoxy mit 1 bis 6 Kohlenstoffatomen oder für den Rest der Formel

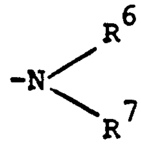

in welcher

$R^6$ und $R^7$ unabhängig voneinander für Wasserstoff, Alkyl mit 1 bis 4 Kohlenstoffatomen oder für gegebenenfalls einfach bis dreifach, gleichartig oder verschieden durch Methyl, Ethyl, Methoxy, Methylthio, Trifluormethyl, Trifluormethoxy, Trifluormethylthio, Fluor, Chlor und/oder Nitro substituiertes Phenyl stehen.

3. Verfahren zur Herstellung von substituierten Phenoxyphenylpropionsäure-Derivaten der Formel

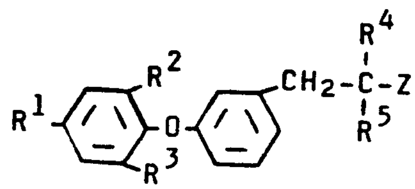

(I)

in welcher

$R^1$ für Halogen, Trihalogenmethylsulfonyl oder Trihalogenmethyl steht,

$R^2$ und $R^3$ unabhängig voneinander für Wasserstoff, Chlor oder Brom stehen,

$R^4$ und $R^5$ unabhängig voneinander für Wasserstoff, Cyano, Thiocyano, Chlor oder Brom stehen und

Z für Cyano oder für den Rest der Formel

$$-CO-Y$$

steht, in welcher

Y für Hydroxy, Halogen, Alkoxy oder für den Rest der Formel

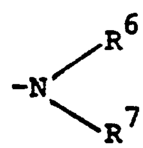

steht, in welcher

$R^6$ und $R^7$ unabhängig voneinander für Wasserstoff, Alkyl mit 1 bis 4 Kohlenstoffatomen sowie für gegebenenfalls einfach bis dreifach substituiertes Phenyl stehen,

dadurch gekennzeichnet, daß man

a) Phenoxyaniline der Formel

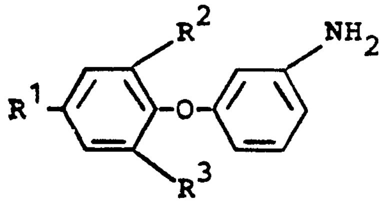

(II)

in welcher

$R^1$, $R^2$ und $R^3$ die oben angegebene Bedeutung haben,

oder Säureadditionssalze von Phenoxyanilinen der Formel (II) mit Diazotierungsmitteln in Gegenwart von Halogenwasserstoffsäuren der Formel

HX (III)

in welcher

X für Chlor oder Brom steht,

und in Gegenwart eines Verdünnungsmittels umsetzt und die dabei entstehenden Diazoniumsalze der Formel

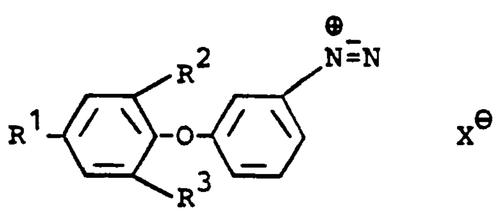

(IV)

in welcher

$R^1$, $R^2$, $R^3$ und X die oben angegebene Bedeutung haben,

mit Acrylsäure-Derivaten der Formel

$$CH_2=\underset{R^8}{C}-Z^1 \qquad (V)$$

in welcher

$R^8$ für Wasserstoff, Chlor oder Brom steht und

$Z^1$ für Cyano oder den Rest der Formel $-CO-Y^1$ steht,

in welcher

$Y^1$ für Hydroxy, Alkoxy oder für den Rest der Formel

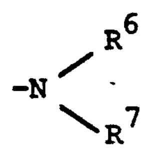

steht, worin

$R^6$ und $R^7$ die oben angegebene Bedeutung haben,

in Gegenwart von Halogenwasserstoffsäuren der Formel

$HR^9$ (VI)

in welcher

$R^9$ für Chlor oder Brom steht,

in Gegenwart eines Katalysators und gegebenenfalls in Gegenwart eines Verdünnungsmittels umsetzt und gegebenenfalls dabei entstehende Ester oder Nitrile in Gegenwart eines Verdünnungsmittels verseift und gegebenenfalls anschließend Phenoxyphenylpropionsäure-Derivate der Formel

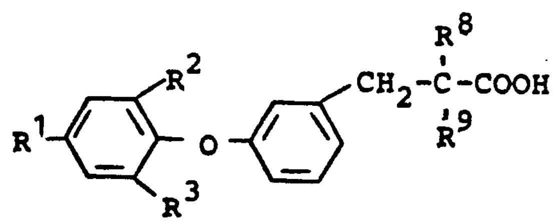

(Ia)

in welcher

$R^1$,$R^2$,$R^3$,$R^8$ und $R^9$ die oben angegebene Bedeutung haben,

gegebenenfalls in Gegenwart eines Verdünnungsmittels halogeniert,

oder

b) substituierte Phenoxyphenylpropionsäure-Derivate der Formel

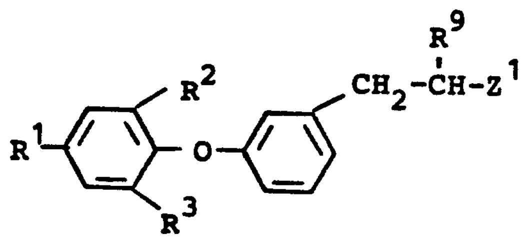

(Ib)

in welcher

$R^1$,$R^2$,$R^3$,$R^9$ und $Z^1$ die oben angegebene Bedeutung haben,

mit Alkalicyaniden oder Alkalirhodaniden gegebenenfalls in Gegenwart eines Verdünnungsmittels umsetzt,

oder

c) substituierte Phenoxyphenylpropionsäure-Derivate der Formel

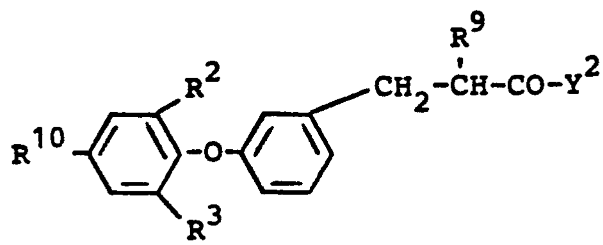

(Ic)

in welcher

$R^2$,$R^3$ und $R^9$ die oben angegebene Bedeutung haben,

$R^{10}$ für Halogen oder Trihalogenmethyl steht und

$Y^2$ für Hydroxy oder Alkoxy steht,

in Gegenwart eines Hydrierungskatalysators mit Wasserstoff gegebenenfalls in Gegenwart eines Verdünnungsmittels umsetzt,

oder

d) Phenoxyphenylpropensäure-Derivate der Formel

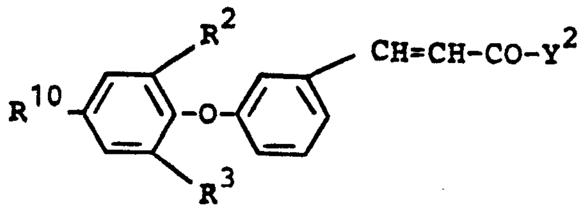

(VII)

in welcher

$R^2$, $R^3$, $R^{10}$ und $Y^2$ die oben angegebene Bedeutung haben,

mit Wasserstoff in Gegenwart eines Hydrierungskatalysators und gegebenenfalls in Gegenwart eines Verdünnungsmittels umsetzt.

4. Herbizide Mittel, gekennzeichnet durch einen Gehalt an mindestens einem substituierten Phenoxyphenylpropionsäure-Derivat der Formel (I).

5. Verfahren zur Bekämpfung von Unkräutern, dadurch gekennzeichnet, daß man substituierte Phenoxyphenylpropionsäure-Derivate der Formel (I) auf die Unkräuter und/oder deren Lebensraum ausbringt.

6. Verwendung von substituierten Phenoxyphenylpropionsäure-Derivaten der Formel (I) zur Bekämpfung von Unkräutern.

7. Verfahren zur Herstellung von herbiziden Mitteln, dadurch gekennzeichnet, daß man substituierte Phenoxyphenylpropionsäure-Derivate der Formel (I) mit Streckmitteln und/oder oberflächenaktiven Stoffen vermischt.

8. Substituiertes Phenoxyphenylpropionsäure-Derivat der Formel

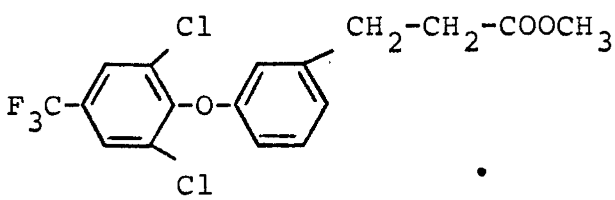

.

9. Substituiertes Phenoxyphenylpropionsäure-Derivat der Formel

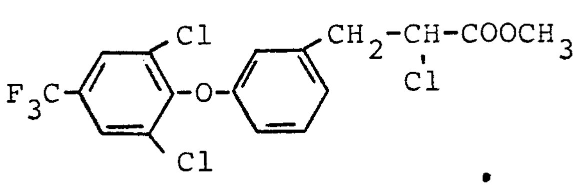

.

1o. Substituiertes Phenoxyphenylpropionsäure-Derivat der Formel